# EUROPÄISCHE PATENTANMELDUNG

(11) **EP 4 375 919 A1**
(43) Veröffentlichungstag der Anmeldung: **29.05.2024**
(21) Anmeldenummer: 22209510.1
(22) Anmeldetag: 25.11.2022
(51) Int. Cl.: G06T 7/00, A61K 49/10

(54) **ERZEUGEN VON KONTRASTVERSTÄRKTEN SYNTHETISCHEN RADIOLOGISCHEN AUFNAHMEN**

(71) Anmelder: Bayer AG, 51373 Leverkusen (DE); Universitätsklinikum Essen, 45147 Essen (DE)
(72) Erfinder: Die Erfindernennung liegt noch nicht vor
(74) Vertreter: BIP Patents

(57) **Zusammenfassung**

Die vorliegende Erfindung befasst sich mit der Erzeugung von synthetischen radiologischen Aufnahmen. Eine solche synthetische radiologischen Aufnahme wird mit Hilfe eines Modells des maschinellen Lernens auf Basis einer CT-Aufnahme eines Untersuchungsbereichs eines Untersuchungsbereichs erzeugt. Die CT-Aufnahme zeigt den Untersuchungsbereich nach der Applikation eines MRT-Kontrastmittels. Das Modell des maschinellen Lernens ist konfiguriert und trainiert, eine synthetische radiologische Aufnahme zu erzeugen, die einer MRT-Aufnahme des Untersuchungsbereichs nach der Applikation des MRT-Kontrastmittels entspricht. Gegenstände der vorliegenden Erfindung sind ein Verfahren zum Trainieren des Modells des maschinellen Lernens, ein computer-implementiertes Verfahren zum Erzeugen der synthetischen radiologischen Aufnahme mittels des trainierten Modells sowie ein Computersystem und ein Computerprogrammprodukt zum Ausführen des Verfahrens zum Erzeugen der synthetischen radiologischen Aufnahme.

## Beschreibung

Die vorliegende Erfindung befasst sich mit der Erzeugung von synthetischen radiologischen Aufnahmen. Eine solche synthetische radiologischen Aufnahme wird mit Hilfe eines Modells des maschinellen Lernens auf Basis einer CT-Aufnahme eines Untersuchungsbereichs eines Untersuchungsobjekts erzeugt. Die CT-Aufnahme zeigt den Untersuchungsbereich nach der Applikation eines MRT-Kontrastmittels. Das Modell des maschinellen Lernens ist konfiguriert und trainiert, eine synthetische radiologische Aufnahme zu erzeugen, die einer MRT-Aufnahme des Untersuchungsbereichs nach der Applikation des MRT-Kontrastmittels entspricht. Gegenstände der vorliegenden Erfindung sind ein Verfahren zum Trainieren des Modells des maschinellen Lernens, ein computer-implementiertes Verfahren zum Erzeugen der synthetischen radiologischen Aufnahme mittels des trainierten Modells sowie ein Computersystem und ein Computerprogrammprodukt zum Ausführen des Verfahrens zum Erzeugen der synthetischen radiologischen Aufnahme.

Die Leber eines Menschen kann von mehreren gutartigen Tumoren betroffen sein, die als zystische oder feste fokale Läsionen im Leberparenchym auftreten können. Die Leber ist ferner anfällig für bösartige Tumoren wie Metastasen von extrahepatischen Krebsarten oder von primären Krebsarten, die ihren Ursprung in der Leber selbst haben. Weltweit sind die beiden häufigsten Arten von bösartigen Lebertumoren Metastasen - insbesondere Darmkrebs-Metastasen - und hepatozelluläre Karzinome (HCC). Fast 20% der Patienten mit Darmkrebs haben zum Zeitpunkt der Diagnose Lebermetastasen, und mehr als 50% der Patienten mit Darmkrebs entwickeln im Verlauf ihrer Krankheit Lebermetastasen. Das hepatozelluläre Karzinom (HCC) ist der häufigste primäre Leberkrebs. Es ist auch die sechsthäufigste Krebserkrankung weltweit und die vierthäufigste Ursache für krebsbedingte Todesfälle.

Die genaue und zuverlässige Erkennung und Charakterisierung von fokalen Leberläsionen in frühen Krankheitsstadien ist von hoher klinischer Relevanz, insbesondere bei Patienten mit einem Risiko für Lebermetastasen oder primären Leberkrebs, da sie für eine angemessene Behandlungsplanung von grundlegender Bedeutung sind und die Eignung für potenziell kurative Behandlungsoptionen bestimmen.

Die Magnetresonanztomographie (MRT) ist von besonderer Bedeutung für die radiologische Untersuchung von Leberläsionen. Sie zeichnet sich durch einen hervorragenden Weichteilkontrast und eine hohe räumliche Auflösung aus, ohne den Patienten ionisierender Strahlung oder iodierten Kontrastmitteln auszusetzen.

Die in der MRT am häufigsten verwendeten Kontrastmittel sind paramagnetische Kontrastmittel auf Gadolinium-Basis. Diese Mittel werden über eine intravenöse (i.v.) Bolusinjektion verabreicht. Ihre kontrastverstärkende Wirkung wird durch das zentrale Gadolinium-Ion (Gd(III), Gd³⁺) im Chelatkomplex vermittelt. Wenn T1-gewichtete Abtastsequenzen in der MRT verwendet werden, führt die durch Gadolinium-Ionen induzierte Verkürzung der Spin-Gitter-Relaxationszeit (T1) von angeregten Atomkernen zu einer Erhöhung der Signalintensität und damit zu einer Erhöhung des Bildkontrasts des untersuchten Gewebes.

Nach ihrem Verteilungsmuster im Gewebe können Gadolinium-basierte Kontrastmittel grob in extrazelluläre und intrazelluläre Kontrastmittel unterteilt werden.

Als extrazelluläre Kontrastmittel werden niedermolekulare, wasserlösliche Verbindungen bezeichnet, die sich nach intravenöser Gabe in den Blutgefäßen und im interstitiellen Raum verteilen. Sie werden nach einer gewissen, vergleichsweise kurzen Zeitspanne der Zirkulation im Blutkreislauf über die Nieren ausgeschieden. Zu den extrazellulären MRT-Kontrastmitteln zählen beispielsweise die Gadolinium-Chelate Gadobutrol (Gadovist^{®}), Gadoteridol (Prohance^{®}), Gadotersäure (Dotarem^{®}), Gadopentetsäure (Magnevist^{®}) und Gadodiamid (Omnican^{®}).

Intrazelluläre Kontrastmittel werden zu gewissen Anteilen in die Zellen von Geweben aufgenommen und anschließend wieder ausgeschieden. Intrazelluläre MRT-Kontrastmittel auf der Basis der Gadoxetsäure zeichnen sich beispielsweise dadurch aus, dass sie anteilig spezifisch von Leberzellen, den Hepatozyten, aufgenommen werden, sich im Funktionsgewebe (Parenchym) anreichern und die Kontraste in gesundem Lebergewebe verstärken, bevor sie anschließend über Galle in die Faeces ausgeschieden werden. Beispiele für derartige Kontrastmittel auf der Basis der Gadoxetsäure sind in US 6,039,931A beschrieben; sie sind kommerziell zum Beispiel unter den Markennamen Primovist^{®} und Eovist^{®} erhältlich. Ein weiteres MRT-Kontrastmittel mit einer geringeren Aufnahme in die Hepatozyten ist Gadobenate Dimeglumine (Multihance^{®}).

Gadoxetat-Dinatrium (GD, Primovist^{®}) gehört zur Gruppe der intrazellulären Kontrastmittel. Es ist zur Verwendung in der MRT der Leber zugelassen, um Läsionen bei Patienten mit bekannter oder vermuteter fokaler Lebererkrankung zu erkennen und zu charakterisieren. GD weist mit seiner lipophilen Ethoxybenzyl-Einheit eine zweiphasige Verteilung auf: zunächst eine Verteilung im intravaskulären und interstitiellen Raum nach Bolusinjektion, gefolgt von einer selektiven Aufnahme durch Hepatozyten. GD wird über die Nieren und den hepatobiliären Weg (50:50 dualer Ausscheidungsmechanismus) in etwa gleichen Mengen unverändert aus dem Körper ausgeschieden. GD wird aufgrund seiner selektiven Anreicherung im gesunden Lebergewebe auch als hepatobiliäres Kontrastmittel bezeichnet.

GD ist in einer Dosis von 0,1 ml / kg Körpergewicht (BW) (0,025 mmol / kg BW Gd) zugelassen. Die empfohlene Verabreichung von GD umfasst eine unverdünnte intravenöse Bolusinjektion mit einer Flussrate von ungefähr 2 ml / Sekunde, gefolgt von einer Spülung der i.v. Kanüle mit einer physiologischen Kochsalzlösung. Ein Standardprotokoll für die Leberbildgebung unter Verwendung von GD besteht aus mehreren Planungs- und Vorkontrastsequenzen. Nach i.v. Bolusinjektion des Kontrastmittels, werden üblicherweise dynamische Aufnahmen während der arteriellen (ungefähr 30 Sekunden nach der Injektion, p.i.), portalvenösen (ungefähr 60 Sekunden p.i.) und der Übergangsphase (ungefähr 2-5 Minuten p.i.) aufgenommen. Die Übergangsphase zeigt typischerweise bereits einen gewissen Anstieg der Lebersignalintensität aufgrund der beginnenden Aufnahme des Mittels in Hepatozyten. Zusätzliche T2-gewichtete und diffusionsgewichtete (DWI) Aufnahmen können nach der dynamischen Phase und vor der späten hepatobiliären Phase erzeugt werden.

Die kontrastverstärkten dynamischen Aufnahmen aus der arteriellen, portalvenösen und der Übergangsphase liefern entscheidende Informationen über die zeitlich variierenden Muster der Läsionsverstärkung (Vaskularisation), die zur Charakterisierung der spezifischen Leberläsion beitragen. Das hepatozelluläre Karzinom, mit seinem typischen arteriellen Phasenhyperenhancement (APHE) und dem Auswaschen (engl.: *washout)* des Kontrastes in der venösen Phase, kann allein anhand seines einzigartigen Vaskularisierungsmuster diagnostiziert werden, das während der dynamischen Phasenbildgebung beobachtet wird, wodurch Patienten vor einer invasiven und möglicherweise riskanten Leberbiopsie geschützt werden.

Auch andere Läsionen lassen sich mittels dynamischer kontrastverstärkter MRT charakterisieren.

In der Diagnose von Leberläsionen hat ein hepatobiliäres Kontrastmittel gegenüber einem extrazellulären Kontrastmittel den Vorteil, dass es eine höhere Sensitivität aufweist und damit insbesondere kleinere Karzinome besser erkennen lässt (siehe z.B.: R.F. Hanna et al.: Comparative 13-year meta-analysis of the sensitivity and positive predictive value of ultrasound, CT, and MRI for detecting hepatocellular carcinoma, Abdom Radiol 2016, 41, 71-90; Y.J. Lee et al.: Hepatocellular carcinoma: diagnostic performance of multidetector CT and MR imaging-a systematic review and meta-analysis, Radiology 2015, 275, 97-109; D.K Owens et al.: High-value, cost-conscious health care: concepts for clinicians to evaluate the benefits, harms, and costs of medical interventions, Ann Intern Med 2011, 154, 174-180).

Die Computertomographie (CT) hat gegenüber der Magnetresonanztomografie den Vorteil, dass während der Erzeugung von CT-Aufnahmen eines Untersuchungsbereichs eines Untersuchungsobjekts chirurgische Eingriffe in dem Untersuchungsbereich möglich sind. Während ein Chirurg also einen Eingriff in dem Untersuchungsbereich vornimmt, kann er den Untersuchungsbereich mit Hilfe der CT bildhaft darstellen.

Möchte ein Chirurg beispielsweise einen Eingriff in der Leber eines Patienten vornehmen, um z.B. eine Biopsie an einer Leberläsion durchzuführen oder einen Tumor zu entfernen, so ist in einer CT-Aufnahme der Leber der Kontrast zwischen einer Leberläsion oder dem Tumor und gesundem Lebergewebe nicht so ausgeprägt wie in einer MRT-Aufnahme nach der Applikation eines hepatobiliären Kontrastmittels. In der CT sind derzeit keine hepatobiliären CT-spezifischen Kontrastmittel bekannt und/oder zugelassen. Es wäre daher wünschenswert, eine radiologische Aufnahme mittels eines Computertomographen erzeugen zu können, in der Leberläsionen ähnlich gut von gesundem Lebergewebe unterschieden werden könnte, wie in einer MRT-Aufnahme nach der Applikation eines hepatobiliären MRT-Kontrastmittels.

Diese Aufgabe und weitere Aufgaben werden durch die Gegenstände der vorliegenden Erfindung gelöst.

Ein erster Gegenstand der vorliegenden Erfindung ist ein computer-implementiertes Verfahren zum Trainieren eines Modells des maschinellen Lernens, umfassend
- Empfangen und/oder Bereitstellen von Trainingsdaten, wobei die Trainingsdaten für jedes Untersuchungsobjekt einer Vielzahl an Untersuchungsobjekten i) eine MRT-Aufnahme eines Untersuchungsbereiches des Untersuchungsobjekts nach der Applikation eines MRT-Kontrastmittels und ii) eine CT-Aufnahme des Untersuchungsbereichs des Untersuchungsobjekts nach der Applikation des MRT-Kontrastmittels umfassen,
- Bereitstellen eines Modells des maschinellen Lernens, wobei das Modell des maschinellen Lernens konfiguriert ist, auf Basis einer CT-Aufnahme, die einen Untersuchungsbereich eines Untersuchungsobjekts nach der Applikation eines MRT-Kontrastmittels repräsentiert, und auf Basis von Modellparametern eine synthetische radiologische Aufnahme zu erzeugen,
- Trainieren des Modells des maschinellen Lernens, wobei das Trainieren für jedes Untersuchungsobjekt der Vielzahl an Untersuchungsobjekten umfasst:
   ∘ Eingeben der CT-Aufnahme in das Modell des maschinellen Lernens
   ∘ Empfangen einer synthetischen radiologischen Aufnahme von dem Modell des maschinellen Lernens
   ∘ Bestimmen einer Abweichung zwischen der synthetischen radiologischen Aufnahme und der MRT-Aufnahme
   ∘ Modifizieren der Modellparameter im Hinblick auf eine Reduzierung der Abweichung
- Speichern und/oder Ausgeben des trainierten Modells des maschinellen Lernens und/oder Übermitteln des trainierten Modells des maschinellen Lernens auf ein separates Computersystem und/oder Verwenden des trainierten Modells des maschinellen Lernens zum Erzeugen einer künstlichen MRT-Aufnahme auf Basis einer neuen gemessenen CT-Aufnahme.

Ein weiterer Gegenstand der vorliegenden Erfindung ist ein computer-implementiertes Verfahren zum Erzeugen einer synthetischen radiologischen Aufnahme umfassend
- Empfangen einer CT-Aufnahme eines Untersuchungsbereichs eines Untersuchungsobjekts nach einer Applikation eines MRT-Kontrastmittels,
- Eingeben der CT-Aufnahmen in ein trainiertes Modell des maschinellen Lernens, wobei das Modell des maschinellen Lernens anhand von Trainingsdaten umfassend, für jedes Untersuchungsobjekt einer Vielzahl an Untersuchungsobjekten, i) eine CT-Aufnahme des Untersuchungsbereichs nach der Applikation des MRT-Kontrastmittels und ii) eine MRT-Aufnahme nach Applikation des MRT-Kontrastmittels, trainiert ist, auf Basis der CT-Aufnahme, eine synthetische radiologische Aufnahme zu erzeugen,
- Empfangen einer synthetischen radiologischen Aufnahme von dem trainierten Modell des maschinellen Lernens,
- Ausgeben und/oder Speichern der synthetischen radiologischen Aufnahme und/oder Übermitteln der synthetischen radiologischen Aufnahme an ein separates Computersystem.

Ein weiterer Gegenstand der vorliegenden Erfindung ist ein Computersystem umfassend:
einen Prozessor; und
einen Speicher, der ein Anwendungsprogramm speichert, das so konfiguriert ist, dass es, wenn es vom Prozessor ausgeführt wird, eine Operation durchführt, wobei die Operation umfasst:
   - Empfangen einer CT-Aufnahme eines Untersuchungsbereichs eines Untersuchungsobjekts nach einer Applikation eines MRT-Kontrastmittels,
   - Eingeben der CT-Aufnahmen in ein trainiertes Modell des maschinellen Lernens, wobei das Modell des maschinellen Lernens anhand von Trainingsdaten umfassend, für jedes Untersuchungsobjekt einer Vielzahl an Untersuchungsobjekten, i) eine CT-Aufnahme des Untersuchungsbereichs nach der Applikation des MRT-Kontrastmittels und ii) eine MRT-Aufnahme nach Applikation des MRT-Kontrastmittels, trainiert ist, auf Basis der CT-Aufnahme, eine synthetische radiologische Aufnahme zu erzeugen,
   - Empfangen einer synthetischen radiologischen Aufnahme von dem trainierten Modell des maschinellen Lernens,
   - Ausgeben und/oder Speichern der synthetischen radiologischen Aufnahme und/oder Übermitteln der synthetischen radiologischen Aufnahme an ein separates Computersystem.

Ein weiterer Gegenstand der vorliegenden Erfindung ist ein Computerprogrammprodukt, das in einen Arbeitsspeicher eines Computersystems geladen werden kann und dort das Computersystem dazu veranlasst, folgende Schritte ausführen:
- Empfangen einer CT-Aufnahme eines Untersuchungsbereichs eines Untersuchungsobjekts nach einer Applikation eines MRT-Kontrastmittels,
- Eingeben der CT-Aufnahmen in ein trainiertes Modell des maschinellen Lernens, wobei das Modell des maschinellen Lernens anhand von Trainingsdaten umfassend, für jedes Untersuchungsobjekt einer Vielzahl an Untersuchungsobjekten, i) eine CT-Aufnahme des Untersuchungsbereichs nach der Applikation des MRT-Kontrastmittels und ii) eine MRT-Aufnahme nach Applikation des MRT-Kontrastmittels, trainiert ist, auf Basis der CT-Aufnahme, eine synthetische radiologische Aufnahme zu erzeugen,
- Empfangen einer synthetischen radiologischen Aufnahme von dem trainierten Modell des maschinellen Lernens,
- Ausgeben und/oder Speichern der synthetischen radiologischen Aufnahme und/oder Übermitteln der synthetischen radiologischen Aufnahme an ein separates Computersystem.

Ein weiterer Gegenstand der vorliegenden Erfindung ist ein MRT-Kontrastmittel zur Verwendung in einem CT-Untersuchungsverfahren umfassend:
- Empfangen einer CT-Aufnahme eines Untersuchungsbereichs eines Untersuchungsobjekts nach einer Applikation eines MRT-Kontrastmittels,
- Eingeben der CT-Aufnahmen in ein trainiertes Modell des maschinellen Lernens, wobei das Modell des maschinellen Lernens anhand von Trainingsdaten umfassend, für jedes Untersuchungsobjekt einer Vielzahl an Untersuchungsobjekten, i) eine CT-Aufnahme des Untersuchungsbereichs nach der Applikation des MRT-Kontrastmittels und ii) eine MRT-Aufnahme nach Applikation des MRT-Kontrastmittels, trainiert ist, auf Basis der CT-Aufnahme, eine synthetische radiologische Aufnahme zu erzeugen,
- Empfangen einer synthetischen radiologischen Aufnahme von dem trainierten Modell des maschinellen Lernens,
- Ausgeben und/oder Speichern der synthetischen radiologischen Aufnahme und/oder Übermitteln der synthetischen radiologischen Aufnahme an ein separates Computersystem.

Ein weiterer Gegenstand der vorliegenden Erfindung ist die Verwendung eines MRT-Kontrastmittels in einem CT-Untersuchungsverfahren umfassend:
- Empfangen einer CT-Aufnahme eines Untersuchungsbereichs eines Untersuchungsobjekts nach einer Applikation eines MRT-Kontrastmittels,
- Eingeben der CT-Aufnahmen in ein trainiertes Modell des maschinellen Lernens, wobei das Modell des maschinellen Lernens anhand von Trainingsdaten umfassend, für jedes Untersuchungsobjekt einer Vielzahl an Untersuchungsobjekten, i) eine CT-Aufnahme des Untersuchungsbereichs nach der Applikation des MRT-Kontrastmittels und ii) eine MRT-Aufnahme nach Applikation des MRT-Kontrastmittels, trainiert ist, auf Basis der CT-Aufnahme, eine synthetische radiologische Aufnahme zu erzeugen,
- Empfangen einer synthetischen radiologischen Aufnahme von dem trainierten Modell des maschinellen Lernens,
- Ausgeben und/oder Speichern der synthetischen radiologischen Aufnahme und/oder Übermitteln der synthetischen radiologischen Aufnahme an ein separates Computersystem.

Ein weiterer Gegenstand der vorliegenden Erfindung ist ein Kit umfassend ein MRT-Kontrastmittel und ein Computerprogrammprodukt wobei das Computerprogrammprodukt ein Computerprogramm umfasst, das in einen Arbeitsspeicher eines Computersystems geladen werden kann und dort das Computersystem dazu veranlasst, folgende Schritte ausführen:
- Empfangen einer CT-Aufnahme eines Untersuchungsbereichs eines Untersuchungsobjekts nach einer Applikation eines MRT-Kontrastmittels,
- Eingeben der CT-Aufnahmen in ein trainiertes Modell des maschinellen Lernens, wobei das Modell des maschinellen Lernens anhand von Trainingsdaten umfassend, für jedes Untersuchungsobjekt einer Vielzahl an Untersuchungsobjekten, i) eine CT-Aufnahme des Untersuchungsbereichs nach der Applikation des MRT-Kontrastmittels und ii) eine MRT-Aufnahme nach Applikation des MRT-Kontrastmittels, trainiert ist, auf Basis der CT-Aufnahme, eine synthetische radiologische Aufnahme zu erzeugen,
- Empfangen einer synthetischen radiologischen Aufnahme von dem trainierten Modell des maschinellen Lernens,
- Ausgeben und/oder Speichern der synthetischen radiologischen Aufnahme und/oder Übermitteln der synthetischen radiologischen Aufnahme an ein separates Computersystem.

Die Erfindung wird nachstehend näher erläutert, ohne zwischen den Erfindungsgegenständen (Trainingsverfahren, Vorhersageverfahren, Computersystem, Computerprogrammprodukt, Verwendung, Kontrastmittel zur Verwendung, Kit) zu unterscheiden. Die nachfolgenden Erläuterungen sollen vielmehr für alle Erfindungsgegenstände in analoger Weise gelten, unabhängig davon, in welchem Kontext (Trainingsverfahren, Vorhersageverfahren, Computersystem, Computerprogrammprodukt, Verwendung, Kontrastmittel zur Verwendung, Kit) sie erfolgen.

Wenn in der vorliegenden Beschreibung oder in den Patentansprüchen Schritte in einer Reihenfolge genannt sind, bedeutet dies nicht zwingend, dass die Erfindung auf die genannte Reihenfolge beschränkt ist. Vielmehr ist denkbar, dass die Schritte auch in einer anderen Reihenfolge oder auch parallel zueinander ausgeführt werden; es sei denn, ein Schritt baut auf einem anderen Schritt auf, was zwingend erforderlich macht, dass der aufbauende Schritt nachfolgend ausgeführt wird (was im Einzelfall aber deutlich wird). Die genannten Reihenfolgen stellen damit bevorzugte Ausführungsformen der Erfindung dar.

Die Erfindung wird an einigen Stellen in Bezug auf Zeichnungen näher erläutert. Dabei sind in den Zeichnungen konkrete Ausführungsformen mit konkreten Merkmalen und Merkmalskombinationen dargestellt, die in erster Linie der Veranschaulichung dienen; die Erfindung soll nicht so verstanden werden, dass sie auf die in den Zeichnungen dargestellten Merkmale und Merkmalskombinationen beschränkt ist. Ferner sollen Aussagen, die bei der Beschreibung der Zeichnungen in Bezug auf Merkmale und Merkmalskombinationen getroffen werden, allgemein gelten, das heißt auch auf andere Ausführungsformen übertragbar und nicht auf die gezeigten Ausführungsformen beschränkt sein.

Die Erfindung stellt Mittel bereit, mit denen eine synthetische radiologische Aufnahme eines Untersuchungsbereichs eines Untersuchungsobjekts erzeugt werden kann.

Das "Untersuchungsobjekt" ist üblicherweise ein Lebewesen, vorzugsweise ein Säugetier, ganz besonders bevorzugt ein Mensch. Das Untersuchungsobjekt wird in dieser Beschreibung auch als Patient bezeichnet.

Der "Untersuchungsbereich" ist ein Teil des Untersuchungsobjekts, zum Beispiel ein Organ oder ein Teil eines Organs wie beispielsweise die Leber, das Gehirn, das Herz, die Niere, die Lunge, der Magen, der Darm oder ein Teil der genannten Organe oder mehrere Organe oder ein anderer Teil des Körpers.

In einer bevorzugten Ausführungsform der vorliegenden Erfindung ist der Untersuchungsbereich die Leber oder ein Teil der Leber eines Menschen.

Der Untersuchungsbereich, auch Aufnahmevolumen (engl.: *field of view,* FOV) genannt, stellt insbesondere ein Volumen dar, welches in radiologischen Aufnahmen abgebildet wird. Der Untersuchungsbereich wird typischerweise durch einen Radiologen, beispielsweise auf einer Übersichtsaufnahme (engl.: *localizer)* festgelegt. Selbstverständlich kann der Untersuchungsbereich alternativ oder zusätzlich auch automatisch, beispielsweise auf Grundlage eines ausgewählten Protokolls, festgelegt werden.

Die synthetische radiologische Aufnahme basiert auf mindestens einer CT-Aufnahme. Die synthetische radiologische Aufnahme repräsentiert einen Untersuchungsbereich eines Untersuchungsobjekts nach der Applikation eines MRT-Kontrastmittels.

Die synthetische radiologische Aufnahme wird mit Hilfe eines trainierten Modells des maschinellen Lernens erzeugt.

Ein "Modell des maschinellen Lernens" kann als eine computerimplementierte Datenverarbeitungsarchitektur verstanden werden. Das Modell kann Eingabedaten empfangen und Ausgabedaten auf der Grundlage dieser Eingabedaten und Modell-Parametern liefern. Das Modell kann durch Training eine Beziehung zwischen den Eingabedaten und den Ausgabedaten erlernen. Beim Training können Modell-Parameter angepasst werden, um eine gewünschte Ausgabe für eine bestimmte Eingabe zu liefern.

Beim Trainieren eines solchen Modells werden dem Modell Trainingsdaten präsentiert, aus denen es lernen kann. Das trainierte Modell des maschinellen Lernens ist das Ergebnis des Trainingsprozesses. Die Trainingsdaten umfassen neben Eingabedaten die korrekten Ausgabedaten (Zieldaten), die das Modell auf Basis der Eingabedaten erzeugen soll. Beim Trainieren werden Muster erkannt, die die Eingabedaten auf die Zieldaten abbilden.

Im Trainingsprozess werden die Eingabedaten der Trainingsdaten in das Modell eingegeben, und das Modell erzeugt Ausgabedaten. Die Ausgabedaten werden mit den Zieldaten verglichen. ModellParameter werden so verändert, dass die Abweichungen zwischen den Ausgabedaten und den Zieldaten auf ein (definiertes) Minimum reduziert werden.

Die Abweichungen können mit Hilfe einer Fehlerfunktion (engl.: *loss function*) quantifiziert werden. Eine solche Fehlerfunktion kann verwendet werden, um einen Fehler (engl.: *loss value)* für ein gegebenes Paar von Ausgabedaten und Zieldaten zu berechnen. Das Ziel des Trainingsprozesses kann darin bestehen, die Parameter des Modells des maschinellen Lernens so zu verändern (anzupassen), dass der Fehler für alle Paare des Trainingsdatensatzes auf ein (definiertes) Minimum reduziert wird.

Handelt es sich bei den Ausgabedaten und den Zieldaten beispielsweise um Zahlen, kann die Fehlerfunktion die absolute Differenz zwischen diesen Zahlen sein. In diesem Fall kann ein hoher absoluter Fehler bedeuten, dass ein oder mehrere Modell-Parameter in hohem Maße geändert werden müssen.

Bei Ausgabedaten in Form von Vektoren können beispielsweise Differenzmetriken zwischen Vektoren wie der mittlere quadratische Fehler, ein Kosinusabstand, eine Norm des Differenzvektors wie ein euklidischer Abstand, ein Tschebyscheff-Abstand, eine Lp-Norm eines Differenzvektors, eine gewichtete Norm oder jede andere Art von Differenzmetrik zweier Vektoren als Fehlerfunktion gewählt werden.

Bei höherdimensionalen Ausgaben, wie z.B. zweidimensionalen, dreidimensionalen oder höherdimensionalen Ausgaben, kann z.B. eine elementweise Differenzmetrik verwendet werden. Alternativ oder zusätzlich können die Ausgabedaten vor der Berechnung eines Fehlerwertes transformiert werden, z.B. in einen eindimensionalen Vektor.

Im vorliegenden Fall wird das Modell des maschinellen Lernens anhand von Trainingsdaten trainiert, ausgehend von mindestens einer CT-Aufnahme des Untersuchungsbereichs des Untersuchungsobjekts nach der Applikation eines MRT-Kontrastmittels, eine synthetische radiologische Aufnahme zu erzeugen, die insbesondere in ihrer Qualität, Kontrastverteilung und Auflösung einer MRT-Aufnahme des Untersuchungsbereichs des Untersuchungsobjekts nach der Applikation eines MRT-Kontrastmittels entspricht. Mit anderen Worten: das Modell des maschinellen Lernens wird trainiert, auf Basis mindestens einer CT-Aufnahme des Untersuchungsbereichs des Untersuchungsobjekts nach der Applikation eines MRT-Kontrastmittels eine künstliche MRT-Aufnahme zu erzeugen (vorherzusagen), die den Untersuchungsbereich des Untersuchungsobjekts nach der Applikation des MRT-Kontrastmittels repräsentiert. Mit anderen Worten: die künstliche radiologische Aufnahme wird auf Basis mindestens einer CT-Aufnahme erzeugt, hat aber eine Kontrastverteilung, die einer MRT-Aufnahme entspricht.

Die Trainingsdaten umfassen, für jedes Untersuchungsobjekt einer Vielzahl von Untersuchungsobjekten mindestens eine CT-Aufnahme und mindestens eine MRT-Aufnahme, wobei die mindestens eine CT-Aufnahme den Untersuchungsbereich des jeweiligen Untersuchungsobjekts nach der Applikation eines MRT-Kontrastmittels repräsentiert, und die mindestens eine MRT-Aufnahme den Untersuchungsbereich des jeweiligen Untersuchungsobjekts nach der Applikation des MRT-Kontrastmittels repräsentiert. Der Untersuchungsbereich ist üblicherweise für alle Untersuchungsobjekte der gleiche. Für unterschiedliche Untersuchungsobjekte können auch unterschiedliche Untersuchungsbereiche ausgewählt werden.

Üblicherweise unterscheiden sich die Kontraste der Strukturen in einer CT-Aufnahme von denen in einer MRT-Aufnahme. Das Modell des maschinellen Lernens wird trainiert, die Kontrastverteilung in einer MRT-Aufnahme auf eine CT-Aufnahme zu übertragen. Damit können Strukturen in einer CT-Aufnahme, die in einer vergleichbaren MRT-Aufnahme kontrastverstärkt dargestellt sind, auch in der CT-Aufnahme deutlicher hervorgehoben werden. Zum Beispiel kann ein hoher Kontrast zwischen gesundem Lebergewebe und Leberläsionen in einer MRT-Aufnahme nach der Applikation eines hepatobiliären MRT-Kontrastmittels auf eine CT-Aufnahme übertragen werden. Mit anderen Worten: dies entspricht der Umwandlung eines hepatobiliären MRT-Kontrastmittels in ein leberspezifisches CT-Kontrastmittel.

Vorzugsweise liegen die mindestens eine CT-Aufnahme und die mindestens eine MRT-Aufnahme im gleichen Format vor. Vorzugsweise haben sie die gleiche Bildgröße (z.B. Zahl der Pixel oder Voxel in den Dimensionen des Bildes). Vorzugsweise handelt es sich jeweils um Repräsentationen des Untersuchungsbereichs in einer Ortsraumdarstellung. Vorzugsweise liegen die mindestens eine CT-Aufnahme und die mindestens eine MRT-Aufnahme in Form von digitalen Bilddaten vor.

Der Begriff "digital" bedeutet, dass die Repräsentationen von einer Maschine, in der Regel einem Computersystem, verarbeitet werden können. Unter "Verarbeitung" werden die bekannten Verfahren zur elektronischen Datenverarbeitung (EDV) verstanden. Ein Beispiel für ein übliches Format für digitale Bilddaten ist das DICOM-Format (DICOM: *Digital Imaging and Communications in Medicine)* - ein offener Standard zur Speicherung und zum Austausch von Informationen im medizinischen Bilddatenmanagement.

Sowohl die mindestens eine CT-Aufnahme als auch die mindestens eine MRT-Aufnahme repräsentieren den Untersuchungsbereich des jeweiligen Untersuchungsobjekts nach der Applikation eines MRT-Kontrastmittels. Üblicherweise ist das Kontrastmittel in beiden Fällen das gleiche. Vorzugsweise aber nicht notwendigerweise wird die gleiche Menge an MRT-Kontrastmittel appliziert.

Die mindestens eine CT-Aufnahme und die mindestens eine MRT-Aufnahme können den Untersuchungsbereich zum gleichen Zeitpunkt nach der Applikation des MRT-Kontrastmittels oder zu verschiedenen Zeitpunkten repräsentieren. Es ist zum Beispiel möglich, dass die mindestens MRT-Aufnahme die Leber oder einen Teil der Leber eines Untersuchungsobjekts in der hepatobiliären Phase nach der Applikation eines hepatobiliären MRT-Kontrastmittels repräsentiert (z.B. 10 bis 20 Minuten nach der Applikation des hepatobiliären MRT-Kontrastmittels), während die mindestens eine CT-Aufnahme die Leber oder den Teil der Leber zu einem früheren Zeitpunkt (z.B. innerhalb der ersten 10 Minuten nach der Applikation des Kontrastmittels) oder zu einem späteren Zeitpunkt (z.B. mehr als 20 Minuten nach der Applikation des Kontrastmittels) nach der Applikation des hepatobiliären Kontrastmittels repräsentiert.

Das MRT-Kontrastmittel ist vorzugsweise ein intrazelluläres Kontrastmittel. Ganz besonders bevorzugt ist das MRT-Kontrastmittel ein hepatobiliäres Kontrastmittel. Unter einem hepatobiliären Kontrastmittel wird ein Kontrastmittel verstanden, das spezifisch von gesunden Leberzellen, den Hepatozyten, aufgenommen wird.

Bespiele für hepatobiliäre Kontrastmittel sind Kontrastmittel auf der Basis der Gadoxetsäure. Sie sind beispielsweise in US 6,039,931A beschrieben. Sie sind kommerziell zum Beispiel unter den Markennamen Primovist^{®} oder Eovist^{®} erhältlich.

Der kontrastverstärkende Effekt von Primovist^{®}/Eovist^{®} wird durch den stabilen Gadoliniumkomplex Gd-EOB-DTPA (Gadolinium-Ethoxybenzyl-Diethylentriamin-Pentaessigsäure) vermittelt. DTPA bildet mit dem paramagnetischen Gadoliniumion einen Komplex, der eine extrem hohe thermodynamische Stabilität aufweist. Der Ethoxybenzylrest (EOB) ist der Vermittler der hepatobiliären Aufnahme des Kontrastmittels.

In einer besonders bevorzugten Ausführungsform handelt es sich bei dem verwendeten Kontrastmittel um einen Stoff oder ein Stoffgemisch mit der Gadoxetsäure oder einem Salz der Gadoxetsäure als kontrastverstärkenden Wirkstoff. Ganz besonders bevorzugt handelt es sich um das Dinatriumsalz der Gadoxetsäure (Gd-EOB-DTPA Dinatrium).

In einer Ausführungsform der vorliegenden Offenbarung handelt es sich bei dem Kontrastmittel um ein Mittel, das einen Gd³⁺-Komplex einer Verbindung der Formel (I) 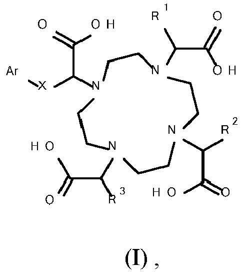 umfasst, wobei
- Ar: eine Gruppe ausgewählt aus 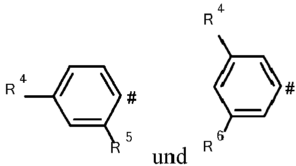 darstellt,
- wobei: # die Anknüpfung zu X darstellt,
- X: eine Gruppe darstellt, die aus CH₂, (CH₂)₂, (CH₂)₃, (CH₂)₄ und ^{∗}-(CH₂)₂-O-CH₂-^{#} ausgewählt wird,
- wobei: ^{∗} die Anknüpfung zu Ar darstellt und ^{#} die Anknüpfung zum Essigsäurerest darstellt,
- R¹, R² and R³: unabhängig voneinander ein Wasserstoffatom oder eine Gruppe ausgewählt aus C₁-C₃-Alkyl, -CH₂OH, -(CH₂)₂OH und -CH₂OCH₃ darstellen,
- R⁴: eine Gruppe ausgewählt aus C₂-C₄-Alkoxy, (H₃C-CH₂)-O-(CH₂)₂-O-, (H₃C-CH₂)-O-(CH₂)₂-O-(CH₂)₂-O- und (H₃C-CH₂)-O-(CH₂)₂-O-(CH₂)₂-O-(CH₂)₂-O- darstellt,
- R⁵: ein Wasserstoffatom darstellt,
- und R⁶: ein Wasserstoffatom darstellt,
oder ein Stereoisomer, Tautomer, Hydrat, Solvat oder Salz davon, oder eine Mischung davon.

In einer Ausführungsform der vorliegenden Offenbarung handelt es sich bei dem Kontrastmittel um ein Mittel, das einen Gd³⁺-Komplex einer Verbindung der Formel (II) 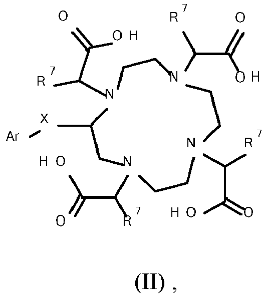 umfasst, wobei
- Ar: eine Gruppe ausgewählt aus 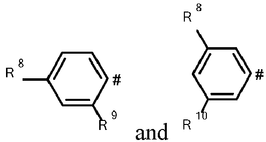 darstellt, wobei # die Anknüpfung zu X darstellt,
X eine Gruppe darstellt, die aus CH₂, (CH₂)₂, (CH₂)₃, (CH₂)₄ and ^{∗}-(CH₂)₂-O-CH₂-^{#} ausgewählt wird, wobei ^{∗} die Anknüpfung zu Ar darstellt und ^{#} die Anknüpfung zum Essigsäurerest darstellt,
R⁷ ein Wasserstoffatom oder eine Gruppe ausgewählt aus C₁-C₃-Alkyl, -CH₂OH, -(CH₂)₂OH und -CH₂OCH₃ darstellt;
R⁸ eine Gruppe ausgewählt aus C₂-C₄-Alkoxy, (H₃C-CH₂O)-(CH₂)₂-O-, (H₃C-CH₂O)-(CH₂)₂-O-(CH₂)₂-O- und (H₃C-CH₂O)-(CH₂)₂-O-(CH₂)₂-O-(CH₂)₂-O- darstellt;
R⁹ und R¹⁰ unabhängig voneinander ein Wasserstoffatom darstellen; oder ein Stereoisomer, Tautomer, Hydrat, Solvat oder Salz davon, oder eine Mischung davon.

Der Begriff "C₁-C₃-Alkyl" bedeutet eine lineare oder verzweigte, gesättigte, einwertige Kohlenwasserstoffgruppe mit 1, 2 oder 3 Kohlenstoffatomen, z.B. Methyl, Ethyl, n-Propyl und Isopropyl. Der Begriff "C₂-C₄-Alkyl" bedeutet eine lineare oder verzweigte, gesättigte, einwertige Kohlenwasserstoffgruppe mit 2, 3 oder 4 Kohlenstoffatomen.

Der Begriff "C₂-C₄-Alkoxy" bedeutet eine lineare oder verzweigte, gesättigte, einwertige Gruppe der Formel (C₂-C₄-Alkyl)-O-, in der der Begriff "C₂-C₄-Alkyl" wie oben definiert ist, z. B. ein Methoxy, Ethoxy, n-Propoxy oder Isopropoxygruppe.

In einer Ausführungsform der vorliegenden Offenbarung handelt es sich bei dem Kontrastmittel um ein Mittel, das Gadolinium 2,2',2"-(10-{1-carboxy-2-[2-(4-ethoxyphenyl)ethoxy]ethyl}-1,4,7,10-tetraazacyclododecan-1,4,7-triyl)triacetat umfasst (siehe z.B. WO2022/194777, Beispiel 1).

In einer Ausführungsform der vorliegenden Offenbarung handelt es sich bei dem Kontrastmittel um ein Mittel, das Gadolinium 2,2',2"-{10-[1-carboxy-2-{4-[2-(2-ethoxyethoxy)ethoxy]phenyl}ethyl]-1,4,7,10-tetraazacyclododecan-1,4,7-triyl}triacetat umfasst (siehe z.B. WO2022/194777, Beispiel 2).

In einer Ausführungsform der vorliegenden Offenbarung handelt es sich bei dem Kontrastmittel um ein Mittel, das Gadolinium 2,2',2"-{10-[(1R)-1-carboxy-2-{4-[2-(2-ethoxyethoxy)ethoxy]phenyl}ethyl]-1,4,7,10-tetraazacyclododecan-1,4,7-triyl}triacetat umfasst (siehe z.B. WO2022/194777, Beispiel 4).

In einer Ausführungsform der vorliegenden Offenbarung handelt es sich bei dem Kontrastmittel um ein Mittel, das Gadolinium (2S,2'S,2"S)-2,2',2"-{10-[(1S)-1-carboxy-4-{4-[2-(2-ethoxyethoxy)ethoxy] phenyl}butyl]-1,4,7,10-tetraazacyclododecan-1,4,7-triyl}tris(3-hydroxypropanoat) umfasst (siehe z.B. WO2022/194777, Beispiel 15).

In einer Ausführungsform der vorliegenden Offenbarung handelt es sich bei dem Kontrastmittel um ein Mittel, das Gadolinium 2,2',2"-{10-[(1S)-4-(4-butoxyphenyl)-1-carboxybutyl]-1,4,7,10-tetraazacyclododecan-1,4,7-triyl}triacetat umfasst (siehe z.B. WO2022/194777, Beispiel 31).

In einer Ausführungsform der vorliegenden Offenbarung handelt es sich bei dem Kontrastmittel um ein Mittel, das Gadolinium-2,2',2"-{(2*S*)-10-(carboxymethyl)-2-[4-(2-ethoxyethoxy)benzyl]-1,4,7,10-tetraazacyclododecane-1,4,7-triyl}triacetat umfasst.

In einer Ausführungsform der vorliegenden Offenbarung handelt es sich bei dem Kontrastmittel um ein Mittel, das Gadolinium-2,2',2"-[10-(carboxymethyl)-2-(4-ethoxybenzyl)-1,4,7,10-tetraazacyclododecane-1,4,7-triyl]triacetat umfasst.

In einer Ausführungsform der vorliegenden Offenbarung handelt es sich bei dem Kontrastmittel um ein Mittel, das Gadolinium(III) 5,8-bis(carboxylatomethyl)-2-[2-(methylamino)-2-oxoethyl]-10-oxo-2,5,8,11-tetraazadodecan-1-carboxylat-Hydrat (auch als Gadodiamid bezeichnet) umfasst.

In einer Ausführungsform der vorliegenden Offenbarung handelt es sich bei dem Kontrastmittel um ein Mittel, das Gadolinium(III) 2-[4-(2-hydroxypropyl)-7,10-bis(2-oxido-2-oxoethyl)-1,4,7,10-tetrazacyclododec-1-yl]acetat (auch als Gadoteridol bezeichnet) umfasst.

In einer Ausführungsform der vorliegenden Offenbarung handelt es sich bei dem Kontrastmittel um ein Mittel, das Gadolinium(III) 2,2',2"-(10-((2R,3S)-1,3,4-trihydroxybutan-2-yl)-1,4,7,10-tetraazacyclododecan-1,4,7-triyl)triacetat (auch als Gadobutrol oder Gd-DO3A-butrol bezeichnet) umfasst.

Die Trainingsdaten können für jedes Untersuchungsobjekt der Vielzahl an Untersuchungsobjekten weitere Daten als Eingabe- und/oder Zieldaten umfassen.

Beispielsweise können die Trainingsdaten neben der mindestens einen CT-Aufnahme, die den Untersuchungsbereich des Untersuchungsobjekt nach der Applikation des MRT-Kontrastmittels repräsentiert, als Eingabedaten mindestens eine weitere CT-Aufnahme umfassen, die den Untersuchungsbereich des Untersuchungsobjekts vor der Applikation des Kontrastmittels, d.h. ohne Kontrastmittel repräsentiert.

Die Trainingsdaten können als Eingabedaten auch mehrere CT-Aufnahmen umfassen, die zu verschiedenen Zeitpunkten vor und/oder nach der Applikation des MRT-Kontrastmittels erzeugt worden sind.

Die Trainingsdaten können als Eingabedaten auch mehrere CT-Aufnahmen umfassen, die unter unterschiedlichen Messbedingungen und/oder unter Verwendung unterschiedlicher Messparameter erzeugt worden sind.

Werden dem Modell des maschinellen Lernens mehrere CT-Aufnahmen als Eingabedaten zugeführt, so erfolgt üblicherweise vorab eine Registrierung.

Eine solche Registrierung (auch "Co-Registrierung" oder Bildregistrierung genannt) ist ein Prozess in der digitalen Bildverarbeitung und dient dazu, zwei oder mehrere Aufnahmen (z.B. Bilder) derselben Szene, oder zumindest ähnlicher Szenen, bestmöglich miteinander in Übereinstimmung zu bringen. Dabei wird eines der Bilder als Referenzbild festgelegt, die anderen werden Objektbilder genannt. Um diese Objektbilder optimal an das Referenzbild anzupassen, wird eine ausgleichende Transformation berechnet. Die zu registrierenden Bilder unterscheiden sich voneinander, weil sie von unterschiedlichen Positionen, zu unterschiedlichen Zeitpunkten oder mit unterschiedlichen Sensoren aufgenommen wurden.

Im Fall der vorliegenden Erfindung können verschiedene CT-Aufnahme zu unterschiedlichen Zeitpunkten aufgenommen worden sein und/oder unterschiedliche Mengen eines Kontrastmittels umfassen.

Das Ziel der Bildregistrierung ist also, diejenige Transformation zu finden, die ein gegebenes Objektbild bestmöglich mit dem Referenzbild in Übereinstimmung bringt. Das Ziel ist, dass nach Möglichkeit jedes Pixel/Voxel eines Bildes denselben Untersuchungsbereich in einem Untersuchungsobjekt repräsentiert wie das Pixel/Voxel eines anderen (co-registrierten) Bildes mit denselben Koordinaten.

Verfahren zur Bildregistrierung sind im Stand der Technik beschrieben (siehe z.B.: E.H. Seeley et al.: Co-registration of multi-modality imaging allows for comprehensive analysis of tumor-induced bone disease, Bone 2014, 61, 208-216; C. Bhushan et al.: Co-registration and distortion correction of diffusion and anatomical images based on inverse contrast normalization, Neuroimage 2015, 15, 115: 269-80; US20200214619; US20090135191; EP3639272A).

Die Trainingsdaten können als Eingabedaten auch weitere Daten umfassen, wie zum Beispiel Informationen zum Untersuchungsobjekt (z.B. Alter, Geschlecht, Körpergröße, Körpergewicht, Body-Mass-Index, Ruheherzfrequenz, Herzfrequenzvariabilität, Körpertemperatur, Informationen über den Lebensstil des Untersuchungsobjekts, wie z.B. Alkoholkonsum, Rauchen und/oder körperliche Betätigung und/oder die Ernährung des Untersuchungsobjekts, medizinische Interventionsparameter wie regelmäßige Medikation, gelegentliche Medikation oder andere frühere oder aktuelle medizinische Interventionen und/oder andere Informationen über frühere und aktuelle Behandlungen des Untersuchungsobjekts und berichtete Gesundheitszustände und/oder Kombinationen davon), Informationen über den Untersuchungsbereich (z.B. Größe und/oder Form des Untersuchungsbereichs und/oder die Lage des Untersuchungsbereichs innerhalb des Untersuchungsobjekts) und/oder Informationen über die mindestens eine CT-Aufnahme (z.B. unter welchen Bedingungen sie erzeugt worden ist und/oder welche Messparameter und/oder Messsequenzen verwendet wurden, um sie zu erzeugen, wann sie erzeugt wurde) und/oder Informationen zu dem verwendeten MRT-Kontrastmittel (z.B. welches Kontrastmittel wurde verwendet, in welcher Menge, wie wurde es verabreicht, wo wurde es verabreicht).

Bei Trainieren des Modells des maschinellen Lernens werden die Eingabedaten dem Modell des maschinellen Lernens zugeführt. Das Modell des maschinellen Lernens ist konfiguriert, auf Basis der Eingabedaten und auf Basis von Modellparametern mindestens eine synthetische radiologische Aufnahme zu erzeugen. Die mindestens eine synthetische radiologische Aufnahme kann mit der mindestens einen MRT-Aufnahme der Trainingsdaten (mit den Zieldaten) verglichen werden.

Mit Hilfe einer Fehlerfunktion können die Abweichungen zwischen der mindestens einen synthetischen radiologischen Aufnahme und der mindestens einen MRT-Aufnahme quantifiziert werden. Geeignete Fehlerfunktionen zur Quantifizierung von Abweichungen zwischen zwei radiologischen Aufnahmen sind zum Beispiel L1-Fehlerfunktion (*L1 loss),* L2-Fehlerfunktion *(L2 loss),* Lp-Fehlerfunktion (*Lp loss),* Strukturähnlichkeitsindexmaß (*structural similarity index measure* (SSIM)), VGG-Fehlerfunktion *(VGG loss),* Wahrnehmungs-Fehlerfunktion (*perceptual loss)* oder eine Kombination der oben genannten Funktionen oder andere Fehlerfunktionen. Weitere Einzelheiten zu Fehlerfunktionen sind z.B. in der wissenschaftlichen Literatur zu finden (siehe z. B.: R. Mechrez *et al*.: The Contextual Loss for Image Transformation with Non-Aligned Data, 2018, arXiv:1803.02077v4; H. Zhao et al.: Loss Functions for Image Restoration with Neural Networks, 2018, arXiv:1511.08861v3).

Mit Hilfe eines Gradientenverfahrens oder eines anderen Optimierungsverfahrens können die Modellparameter so modifiziert werden, dass der Fehler für alle Paare umfassend die synthetische radiologische Aufnahme (Ausgabedaten) und die mindestens eine MRT-Aufnahme (Zieldaten) reduziert und vorzugsweise unter einen vordefinierten Schwellenwert fällt und/oder auf ein definiertes Minimum reduziert wird.

Ist das Modell auf Basis einer Vielzahl an Paaren aus Eingabedaten und Zieldaten trainiert worden und hat der Fehler (L) für alle Paare aus Ausgabedaten und Zieldaten ein definiertes Minimum erreicht, können das trainierte Modell des maschinellen Lernens und/oder die modifizierten Modellparameter in einem Datenspeicher gespeichert und/oder an ein separates Computersystem übermittelt und/oder zur Vorhersage genutzt werden.

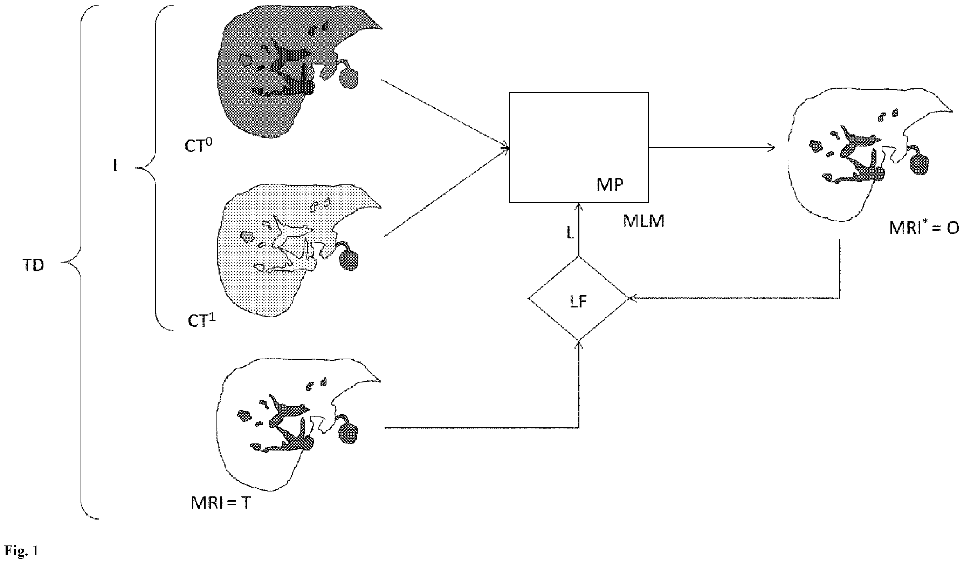

Fig. 1 zeigt beispielhaft und schematisch eine Ausführungsform des Verfahrens zum Trainieren des Modells des maschinellen Lernens.

Das Modell des maschinellen Lernens wird mit Hilfe von Trainingsdaten (TD) trainiert. Üblicherweise umfassen die Trainingsdaten für jedes Untersuchungsobjekt einer Vielzahl von Untersuchungsobjekten Eingabedaten und Zieldaten. In Fig. 1 ist nur ein Datensatz eines Untersuchungsobjekts gezeigt, der aus den Eingabedaten (I) und den Zieldaten (T) besteht. Die Eingabedaten (I) bestehen im vorliegenden Beispiel aus mindestens einer CT-Aufnahme (CT¹), die einen Teil einer Leber eines Untersuchungsobjekts zu einem Zeitpunkt nach der Applikation eines hepatobiliären MRT-Kontrastmittels repräsentiert, und aus mindestens einer nativen CT-Aufnahme (CT⁰), die die Leber des Untersuchungsobjekts ohne ein Kontrastmittel repräsentiert. Wie beschrieben ist die Verwendung von einer nativen CT-Aufnahme optional. Wie beschrieben können die Eingabedaten weitere Daten umfassen. Die Zieldaten (T) bestehen im vorliegenden Beispiel aus einer MRT-Aufnahme (MRI), die den Teil der Leber zu einem Zeitpunkt in der hepatobiliären Phase nach der Applikation des MRT-Kontrastmittels repräsentiert.

Die Eingabedaten (I) werden dem Modell des maschinellen Lernens (MLM) zugeführt. Das Modell des maschinellen Lernens (MLM) ist konfiguriert, auf Basis der Eingabedaten (I) und auf Basis von Modellparametern (MP) Ausgabedaten (O) zu erzeugen. Die Ausgabedaten (O) stellen eine vorhergesagte MRT-Aufnahme (MRI^{∗}) dar, die den Teil der Leber des Untersuchungsobjekts zu einem Zeitpunkt in der hepatobiliären Phase nach der Applikation des hepatobiliären Kontrastmittels repräsentiert. Insbesondere bezüglich ihrer Qualität, Kontrastverteilung und Auflösung entsprechen die Ausgabedaten (O) einer MRT-Aufnahme des Untersuchungsbereichs des Untersuchungsobjekts nach der Applikation des hepatobiliären MRT-Kontrastmittels.

Mittels einer Fehlerfunktion (LF) werden Abweichungen zwischen der vorhergesagten MRT-Aufnahme (MRI^{∗}) und der MRT-Aufnahme (MRI) der Trainingsdaten quantifiziert. Für jedes Paar aus Ausgabedaten und Zieldaten kann ein Fehler (L) berechnet werden. Der Fehler (L) kann genutzt werden, um Modellparameter (MP) zu modifizieren, so dass der Fehler (L) auf ein definiertes Minimum reduziert wird.

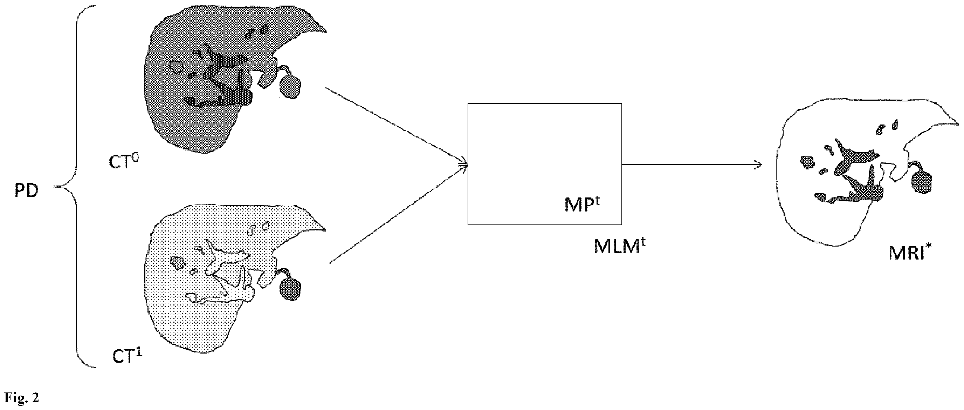

Ist das Modell auf Basis einer Vielzahl an Paaren aus Eingabedaten und Zieldaten trainiert worden und hat der Fehler (L) für alle Paare ein definiertes Minimum erreicht, kann das trainierte Modell des maschinellen Lernens zur Vorhersage genutzt werden. Dies ist beispielhaft und schematisch in Fig. 2 gezeigt.

Fig. 2 zeigt beispielhaft und schematisch eine Ausführungsform des Verfahrens zur Erzeugung einer synthetischen radiologischen Aufnahme, in diesem Fall die Erzeugung einer künstlichen MRT-Aufnahme einer Leber oder eines Teils der Leber eines Patienten, wobei die MRT-Aufnahme die Leber oder den Teil der Leber in einer hepatobiliären Phase nach der Applikation eines hepatobiliären Kontrastmittels darstellt.

Die Vorhersage erfolgt mit Hilfe eines trainierten Modells des maschinellen Lernens (MLM^{t}). Das Modell des maschinellen Lernens kann beispielsweise wie in Bezug zu Fig. 1 beschrieben trainiert worden sein.

Die Vorhersage erfolgt auf Basis von Patientendaten (PD). Die Patientendaten (PD) umfassen mindestens eine CT-Aufnahme (CT¹), die eine Leber oder einen Teil einer Leber eines Untersuchungsobjekts nach der Applikation des hepatobiliären Kontrastmittels repräsentiert. Im vorliegenden Beispiel umfassen die Patientendaten (PD) ferner mindestens eine native CT-Aufnahme (CT⁰). Die mindestens eine native MRT-Aufnahme (CT⁰) ist optional und zeigt die Leber oder den Teil der Leber ohne ein Kontrastmittel. Die Patientendaten können noch weitere Daten umfassen, insbesondere dann, wenn auch das Training des Modells des maschinellen Lernens auf Basis weiterer Eingabedaten erfolgt ist.

Die Patientendaten (PD) werden dem trainierten Modell des maschinellen Lernens (MLM^{t}) zugeführt. Das Modell des maschinellen Lernens (MLM^{t}) ist konfiguriert und trainiert, auf Basis der Patientendaten (PD) und auf Basis der im Rahmen des Trainings modifizierten (gelernten) Modellparameter (MP^{t}) eine synthetische radiologische Aufnahme zu erzeugen. Die synthetische radiologische Aufnahme entspricht insbesondere bezüglich ihrer Qualität, Kontrastverteilung und Auflösung einer MRT-Aufnahme, die die Leber oder den Teil der Leber des Untersuchungsobjekts in der hepatobiliären Phase nach der Applikation des hepatobiliären Kontrastmittels darstellt.

Die Ausgabe der vorhergesagten MRT-Aufnahme (MRI^{∗}) kann auf unterschiedlicher Weise erfolgen, die vorhergesagte MRT-Aufnahme (MRI^{∗}) kannauf einem Monitor angezeigt, auf einem Drucker ausgedruckt, auf einem Datenspeicher gespeichert und/oder an ein separates Computersystem übermittelt werden.

Das Modell des maschinellen Lernens kann beispielsweise ein künstliches neuronales Netz sein oder ein solches umfassen.

Ein künstliches neuronales Netzwerk umfasst mindestens drei Schichten von Verarbeitungselementen: eine erste Schicht mit Eingangsneuronen (Knoten), eine *N*-te Schicht mit mindestens einem Ausgangsneuron (Knoten) und *N*-2 innere Schichten, wobei *N* eine natürliche Zahl und größer als 2 ist.

Die Eingangsneuronen dienen zum Empfangen der Eingangsdaten, insbesondere der mindestens einen CT-Aufnahme einer Leber oder eines Teils der Leber eines Untersuchungsobjekts und optional mindestens einer nativen CT-Aufnahme. Üblicherweise gibt es ein Eingangsneuron für jedes Pixel oder Voxel einer CT-Aufnahme. Es können zusätzliche Eingangsneuronen für zusätzliche Eingangswerte (z.B. Informationen zum Untersuchungsbereich, zum Untersuchungsobjekt, zu Bedingungen, die bei der Erzeugung der CT-Aufnahmen herrschten, Informationen zu dem Zustand, den die CT-Aufnahme repräsentiert, und/oder Informationen zu dem Zeitpunkt oder der Zeitspanne zu/in der die CT-Aufnahme erzeugt worden ist) vorhanden sein.

Die Ausgangsneuronen können dazu dienen, eine vorhergesagte MRT-Aufnahme, die den Untersuchungsbereich in der hepatobiliären Phase repräsentiert, auszugeben.

Die Verarbeitungselemente der Schichten zwischen den Eingangsneuronen und den Ausgangsneuronen sind in einem vorbestimmten Muster mit vorbestimmten Verbindungsgewichten miteinander verbunden.

Vorzugsweise handelt es sich bei dem künstlichen neuronalen Netz um ein so genanntes Convolutional Neural Network (kurz: CNN) oder es umfasst ein solches.

Ein CNN besteht üblicherweise im Wesentlichen aus Filtern (Convolutional Layer) und Aggregations-Schichten (Pooling Layer), die sich abwechselnd wiederholen, und am Ende aus einer Schicht oder mehreren Schichten von "normalen" vollständig verbundenen Neuronen (Dense / Fully Connected Layer).

Das Trainieren des neuronalen Netzes kann beispielsweise mittels eines Backpropagation-Verfahrens durchgeführt werden. Dabei wird für das Netz eine möglichst zuverlässige Vorhersage der zweiten MRT-Aufnahme angestrebt. Die Qualität der Vorhersage wird durch eine Fehlerfunktion beschrieben. Das Ziel ist die Minimierung der Fehlerfunktion. Das Einlernen eines künstlichen neuronalen Netzes erfolgt bei dem Backpropagation-Verfahren durch die Änderung der Verbindungsgewichte.

Im trainierten Zustand enthalten die Verbindungsgewichte zwischen den Verarbeitungselementen Informationen bezüglich des Zusammenhangs zwischen der mindestens einen CT-Aufnahme sowie optional der mindestens einen nativen CT-Aufnahme und der MRT-Aufnahme, die für Vorhersagezwecke verwendet werden können.

Eine Kreuzvalidierungsmethode kann verwendet werden, um die Daten in Trainings- und Validierungsdatensätze aufzuteilen. Der Trainingsdatensatz wird beim Backpropagation-Training der Netzwerkgewichte verwendet. Der Validierungsdatensatz wird verwendet, um zu überprüfen, mit welcher Vorhersagegenauigkeit sich das trainierte Netzwerk auf unbekannte Daten anwenden lässt.

Das künstliche neuronale Netzwerk kann eine Autoencoder-Architektur aufweisen; z.B. kann das künstliche neuronale Netzwerk eine Architektur wie das U-Net aufweisen (siehe z.B. O. Ronneberger et al.: U-net: Convolutional networks for biomedical image segmentation, International Conference on Medical image computing and computer-assisted intervention, Seiten 234-241, Springer, 2015, https://doi.org/10.1007/978-3-319-24574-4_28).

Das künstliche neuronale Netzwerk kann ein *Generative Adversarial Network* (GAN) sein (siehe z.B. M.-Y. Liu et al.: Generative Adversarial Networks for Image and Video Synthesis: Algorithms and Applications, arXiv:2008.02793; J. Henry et al.: Pix2Pix GAN for Image-to-Image Translation, DOI: 10.13140/RG.2.2.32286.66887).

Das künstliche neuronale Netzwerk kann ein rekurrentes neuronales Netzwerk sein oder ein solches umfassen. Als rekurrente bzw. rückgekoppelte neuronale Netzwerke bezeichnet man neuronale Netzwerke, die sich im Gegensatz zu den Feedforward-Netzen durch Verbindungen von Neuronen einer Schicht zu Neuronen derselben oder einer vorangegangenen Schicht auszeichnen. Das künstliche neuronale Netzwerk kann beispielsweise ein *Long short-term memory* (LSTM) umfassen (siehe z.B. Y. Gao et al.: Fully convolutional structured LSTM networks for joint 4D medical image segmentation, DOI: 10.1109/ISBI.2018.8363764).

Das künstliche neuronale Netz kann ein Transfomer-Netzwerk sein (siehe z.B. D. Karimi et al.: Convolution-Free Medical Image Segmentation using Transformers, arXiv:2102.13645 [eess.IV]).

Die Erfindung kann ganz oder teilweise mit Hilfe eines Computersystems ausgeführt werden.

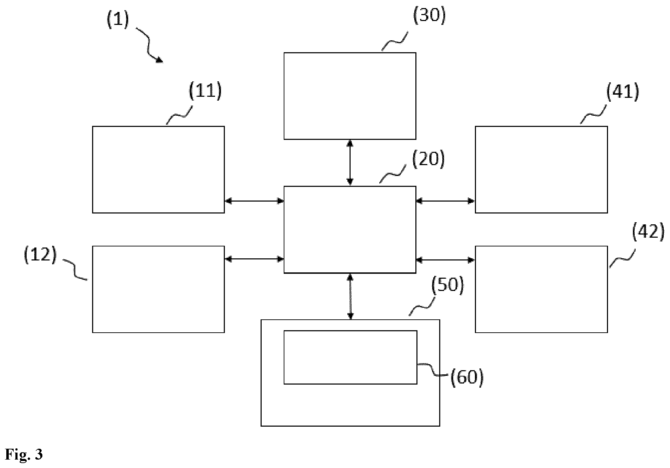

Fig. 3 zeigt beispielhaft und schematisch eine Ausführungsform des erfindungsgemäßen Computersystems.

Das Computersystem (1) umfasst eine Verarbeitungseinheit (20), die mit einem Speicher (50) verbunden ist.

Die Verarbeitungseinheit (20) (engl.: *processing unit)* kann einen oder mehrere Prozessoren allein oder in Kombination mit einem oder mehreren Speichern umfassen. Bei der Verarbeitungseinheit (20) kann es sich um gewöhnliche Computerhardware handeln, die in der Lage ist, Informationen wie z.B. digitale Bildaufnahmen, Computerprogramme und/oder andere digitale Informationen zu verarbeiten. Die Verarbeitungseinheit (20) besteht üblicherweise aus einer Anordnung elektronischer Schaltungen, von denen einige als integrierter Schaltkreis oder als mehrere miteinander verbundene integrierte Schaltkreise (ein integrierter Schaltkreis wird manchmal auch als "Chip" bezeichnet) ausgeführt sein können. Die Verarbeitungseinheit (20) kann konfiguriert sein, Computerprogramme auszuführen, die in einem Arbeitsspeicher der Verarbeitungseinheit (20) oder im Speicher (50) desselben oder eines anderen Computersystems gespeichert sein können.

Der Speicher (50) kann eine gewöhnliche Computerhardware sein, die in der Lage ist, Informationen wie z.B. digitale Bildaufnahmen (z.B. Repräsentationen des Untersuchungsbereichs), Daten, Computerprogramme und/oder andere digitale Informationen entweder vorübergehend und/oder dauerhaft zu speichern. Der Speicher (50) kann einen flüchtigen und/oder nichtflüchtigen Speicher umfassen und kann fest eingebaut oder entfernbar sein. Beispiele für geeignete Speicher sind RAM (Random Access Memory), ROM (Read-Only Memory), eine Festplatte, ein Flash-Speicher, eine austauschbare Computerdiskette, eine optische Disc, ein Magnetband oder eine Kombination der oben genannten. Zu den optischen Discs können Compact Discs mit Nur-Lese-Speicher (CD-ROM), Compact Discs mit Lese-/Schreibfunktion (CD-R/W), DVDs, Blu-ray-Discs und ähnliche gehören.

Zusätzlich zum Speicher (50) kann die Verarbeitungseinheit (20) auch mit einer oder mehreren Schnittstellen (11, 12, 30, 41, 42) verbunden sein, um Informationen anzuzeigen, zu übertragen und/oder zu empfangen. Die Schnittstellen können eine oder mehrere Kommunikationsschnittstellen (11, 12) und/oder eine oder mehrere Benutzerschnittstellen (30, 41, 42) umfassen. Die eine oder mehrere Kommunikationsschnittstellen können so konfiguriert sein, dass sie Informationen senden und/oder empfangen, z.B. zu und/oder von einer MRT-Scanner, einem CT-Scanner, einer Ultraschallkamera, anderen Computersystemen, Netzwerken, Datenspeichern oder dergleichen. Die eine oder mehrere Kommunikationsschnittstellen können so konfiguriert sein, dass sie Informationen über physische (verdrahtete) und/oder drahtlose Kommunikationsverbindungen übertragen und/oder empfangen. Die eine oder die mehreren Kommunikationsschnittstellen können eine oder mehrere Schnittstellen für die Verbindung mit einem Netzwerk enthalten, z.B. unter Verwendung von Technologien wie Mobiltelefon, Wi-Fi, Satellit, Kabel, DSL, Glasfaser und/oder dergleichen. In einigen Beispielen können die eine oder die mehreren Kommunikationsschnittstellen eine oder mehrere Nahbereichskommunikationsschnittstellen umfassen, die so konfiguriert sind, dass sie Geräte mit Nahbereichskommunikationstechnologien wie NFC, RFID, Bluetooth, Bluetooth LE, ZigBee, Infrarot (z. B. IrDA) oder Ähnlichem verbinden.

Die Benutzerschnittstellen können eine Anzeige (30) umfassen. Eine Anzeige (30) kann so konfiguriert sein, dass sie einem Benutzer Informationen anzeigt. Geeignete Beispiele hierfür sind eine Flüssigkristallanzeige (LCD), eine Leuchtdiodenanzeige (LED), ein Plasmabildschirm (PDP) oder Ähnliches. Die Benutzereingabeschnittstelle(n) (41, 42) kann/können verdrahtet oder drahtlos sein und kann/können so konfiguriert sein, dass sie Informationen von einem Benutzer in das Computersystem (1) empfängt/empfangen, z.B. zur Verarbeitung, Speicherung und/oder Anzeige. Geeignete Beispiele für Benutzereingabeschnittstellen sind ein Mikrofon, ein Bild- oder Videoaufnahmegerät (z.B. eine Kamera), eine Tastatur oder ein Tastenfeld, ein Joystick, eine berührungsempfindliche Oberfläche (getrennt von einem Touchscreen oder darin integriert) oder ähnliches. In einigen Beispielen können die Benutzerschnittstellen eine automatische Identifikations- und Datenerfassungstechnologie (AIDC) für maschinenlesbare Informationen enthalten. Dazu können Barcodes, Radiofrequenz-Identifikation (RFID), Magnetstreifen, optische Zeichenerkennung (OCR), Karten mit integrierten Schaltkreisen (ICC) und ähnliches gehören. Die Benutzerschnittstellen können ferner eine oder mehrere Schnittstellen für die Kommunikation mit Peripheriegeräten wie Druckern und dergleichen umfassen.

Ein oder mehrere Computerprogramme (60) können im Speicher (50) gespeichert sein und von der Verarbeitungseinheit (20) ausgeführt werden, die dadurch programmiert wird, die in dieser Beschreibung beschriebenen Funktionen zu erfüllen. Das Abrufen, Laden und Ausführen von Anweisungen des Computerprogramms (60) kann sequenziell erfolgen, so dass jeweils ein Befehl abgerufen, geladen und ausgeführt wird. Das Abrufen, Laden und/oder Ausführen kann aber auch parallel erfolgen.

In dem Speicher (50) kann auch das erfindungsgemäße Modell des maschinellen Lernens gespeichert sein.

Das erfindungsgemäße System kann als Laptop, Notebook, Netbook und/der Tablet-PC ausgeführt sein, es kann auch ein Bestandteil eines MRT-Scanners, eines CT-Scanners oder eines Ultraschalldiagnosegeräts sein.

## Patentansprüche

1. Computer-implementiertes Verfahren zum Trainieren eines Modells des maschinellen Lernens, umfassend
- Empfangen und/oder Bereitstellen von Trainingsdaten, wobei die Trainingsdaten für jedes Untersuchungsobjekt einer Vielzahl an Untersuchungsobjekten i) eine MRT-Aufnahme eines Untersuchungsbereiches des Untersuchungsobjekts nach der Applikation eines MRT-Kontrastmittels und ii) eine CT-Aufnahme des Untersuchungsbereichs des Untersuchungsobjekts nach der Applikation des MRT-Kontrastmittels umfassen,
- Bereitstellen eines Modells des maschinellen Lernens, wobei das Modell des maschinellen Lernens konfiguriert ist, auf Basis einer CT-Aufnahme, die einen Untersuchungsbereich eines Untersuchungsobjekts nach der Applikation eines MRT-Kontrastmittels repräsentiert, und auf Basis von Modellparametern eine synthetische radiologische Aufnahme erzeugen,
- Trainieren des Modells des maschinellen Lernens, wobei das Trainieren für jedes Untersuchungsobjekt der Vielzahl an Untersuchungsobjekten umfasst:
∘ Eingeben der CT-Aufnahme in das Modell des maschinellen Lernens
∘ Empfangen einer synthetischen radiologischen Aufnahme von dem Modell des maschinellen Lernens
∘ Bestimmen einer Abweichung zwischen der synthetischen radiologischen Aufnahme und der MRT-Aufnahme
∘ Modifizieren der Modellparameter im Hinblick auf eine Reduzierung der Abweichung
- Speichern und/oder Ausgeben des trainierten Modells des maschinellen Lernens und/oder Übermitteln des trainierten Modells des maschinellen Lernens auf ein separates Computersystem und/oder Verwenden des trainierten Modells des maschinellen Lernens zum Erzeugen einer künstlichen MRT-Aufnahme auf Basis einer neuen gemessenen CT-Aufnahme.

2. Computer-implementiertes Verfahren zum Erzeugen einer synthetischen radiologischen Aufnahme umfassend
- Empfangen einer CT-Aufnahme eines Untersuchungsbereichs eines Untersuchungsobjekts nach einer Applikation eines MRT-Kontrastmittels,
- Eingeben der CT-Aufnahmen in ein trainiertes Modell des maschinellen Lernens, wobei das Modell des maschinellen Lernens anhand von Trainingsdaten umfassend, für jedes Untersuchungsobjekt einer Vielzahl an Untersuchungsobjekten, i) eine CT-Aufnahme des Untersuchungsbereichs nach der Applikation des MRT-Kontrastmittels und ii) eine MRT-Aufnahme nach Applikation des MRT-Kontrastmittels, trainiert ist, auf Basis der CT-Aufnahme, eine synthetische radiologische Aufnahme zu erzeugen,
- Empfangen einer synthetischen radiologischen Aufnahme von dem trainierten Modell des maschinellen Lernens,
- Ausgeben und/oder Speichern der synthetischen radiologischen Aufnahme und/oder Übermitteln der synthetischen radiologischen Aufnahme an ein separates Computersystem.

3. Verfahren nach einem der Ansprüche 1 oder 2, wobei das Untersuchungsobjekt ein Lebewesen, vorzugsweise ein Säugetier, ganz besonders bevorzugt ein Mensch ist.

4. Computersystem umfassend:
einen Prozessor; und
einen Speicher, der ein Anwendungsprogramm speichert, das so konfiguriert ist, dass es, wenn es vom Prozessor ausgeführt wird, eine Operation durchführt, wobei die Operation umfasst:
- Empfangen einer CT-Aufnahme eines Untersuchungsbereichs eines Untersuchungsobjekts nach einer Applikation eines MRT-Kontrastmittels,
- Eingeben der CT-Aufnahmen in ein trainiertes Modell des maschinellen Lernens, wobei das Modell des maschinellen Lernens anhand von Trainingsdaten umfassend, für jedes Untersuchungsobjekt einer Vielzahl an Untersuchungsobjekten, i) eine CT-Aufnahme des Untersuchungsbereichs nach der Applikation des MRT-Kontrastmittels und ii) eine MRT-Aufnahme nach Applikation des MRT-Kontrastmittels, trainiert ist, auf Basis der CT-Aufnahme, eine synthetische radiologische Aufnahme zu erzeugen,
- Empfangen einer synthetischen radiologischen Aufnahme von dem trainierten Modell des maschinellen Lernens,
- Ausgeben und/oder Speichern der synthetischen radiologischen Aufnahme und/oder Übermitteln der synthetischen radiologischen Aufnahme an ein separates Computersystem.

5. Computerprogrammprodukt, das in einen Arbeitsspeicher eines Computersystems geladen werden kann und dort das Computersystem dazu veranlasst, folgende Schritte ausführen:
- Empfangen einer CT-Aufnahme eines Untersuchungsbereichs eines Untersuchungsobjekts nach einer Applikation eines MRT-Kontrastmittels,
- Eingeben der CT-Aufnahmen in ein trainiertes Modell des maschinellen Lernens, wobei das Modell des maschinellen Lernens anhand von Trainingsdaten umfassend, für jedes Untersuchungsobjekt einer Vielzahl an Untersuchungsobjekten, i) eine CT-Aufnahme des Untersuchungsbereichs nach der Applikation des MRT-Kontrastmittels und ii) eine MRT-Aufnahme nach Applikation des MRT-Kontrastmittels, trainiert ist, auf Basis der CT-Aufnahme, eine synthetische radiologische Aufnahme zu erzeugen,
- Empfangen einer synthetischen radiologischen Aufnahme von dem trainierten Modell des maschinellen Lernens,
- Ausgeben und/oder Speichern der synthetischen radiologischen Aufnahme und/oder Übermitteln der synthetischen radiologischen Aufnahme an ein separates Computersystem.

6. MRT-Kontrastmittel zur Verwendung in einem CT-Untersuchungsverfahren umfassend:
- Empfangen einer CT-Aufnahme eines Untersuchungsbereichs eines Untersuchungsobjekts nach einer Applikation eines MRT-Kontrastmittels,
- Eingeben der CT-Aufnahmen in ein trainiertes Modell des maschinellen Lernens, wobei das Modell des maschinellen Lernens anhand von Trainingsdaten umfassend, für jedes Untersuchungsobjekt einer Vielzahl an Untersuchungsobjekten, i) eine CT-Aufnahme des Untersuchungsbereichs nach der Applikation des MRT-Kontrastmittels und ii) eine MRT-Aufnahme nach Applikation des MRT-Kontrastmittels, trainiert ist, auf Basis der CT-Aufnahme, eine synthetische radiologische Aufnahme zu erzeugen,
- Empfangen einer synthetischen radiologischen Aufnahme von dem trainierten Modell des maschinellen Lernens,
- Ausgeben und/oder Speichern der synthetischen radiologischen Aufnahme und/oder Übermitteln der synthetischen radiologischen Aufnahme an ein separates Computersystem.

7. Verwendung eines MRT-Kontrastmittels in einem CT-Untersuchungsverfahren umfassend:
- Empfangen einer CT-Aufhahme eines Untersuchungsbereichs eines Untersuchungsobjekts nach einer Applikation eines MRT-Kontrastmittels,
- Eingeben der CT-Aufnahmen in ein trainiertes Modell des maschinellen Lernens, wobei das Modell des maschinellen Lernens anhand von Trainingsdaten umfassend, für jedes Untersuchungsobjekt einer Vielzahl an Untersuchungsobjekten, i) eine CT-Aufnahme des Untersuchungsbereichs nach der Applikation des MRT-Kontrastmittels und ii) eine MRT-Aufnahme nach Applikation des MRT-Kontrastmittels, trainiert ist, auf Basis der CT-Aufnahme, eine synthetische radiologische Aufnahme zu erzeugen,
- Empfangen einer synthetischen radiologischen Aufnahme von dem trainierten Modell des maschinellen Lernens,
- Ausgeben und/oder Speichern der synthetischen radiologischen Aufnahme und/oder Übermitteln der synthetischen radiologischen Aufnahme an ein separates Computersystem.

8. Kit umfassend ein MRT-Kontrastmittel und ein Computerprogrammprodukt wobei das Computerprogrammprodukt ein Computerprogramm umfasst, das in einen Arbeitsspeicher eines Computersystems geladen werden kann und dort das Computersystem dazu veranlasst, folgende Schritte ausführen:
- Empfangen einer CT-Aufnahme eines Untersuchungsbereichs eines Untersuchungsobjekts nach einer Applikation eines MRT-Kontrastmittels,
- Eingeben der CT-Aufnahmen in ein trainiertes Modell des maschinellen Lernens, wobei das Modell des maschinellen Lernens anhand von Trainingsdaten umfassend, für jedes Untersuchungsobjekt einer Vielzahl an Untersuchungsobjekten, i) eine CT-Aufnahme des Untersuchungsbereichs nach der Applikation des MRT-Kontrastmittels und ii) eine MRT-Aufnahme nach Applikation des MRT-Kontrastmittels, trainiert ist, auf Basis der CT-Aufnahme, eine synthetische radiologische Aufnahme zu erzeugen,
- Empfangen einer synthetischen radiologischen Aufnahme von dem trainierten Modell des maschinellen Lernens,
- Ausgeben und/oder Speichern der synthetischen radiologischen Aufnahme und/oder Übermitteln der synthetischen radiologischen Aufnahme an ein separates Computersystem.

9. Verfahren, Computersystem, Computerprogrammprodukt, MRT-Kontrastmittel, Verwendung oder Kit nach einem der Ansprüche 1 bis 8, wobei das Trainieren des Modells des maschinellen Lernens für jedes Untersuchungsobjekt der Vielzahl an Untersuchungsobjekten umfasst:
∘ Eingeben der CT-Aufnahme in das Modell des maschinellen Lernens
∘ Empfangen einer synthetischen radiologischen Aufnahme von dem Modell des maschinellen Lernens
∘ Bestimmen einer Abweichung zwischen der synthetischen radiologischen Aufnahme und der MRT-Aufnahme
∘ Modifizieren der Modellparameter im Hinblick auf eine Reduzierung der Abweichung.

10. MRT-Kontrastmittel zur Verwendung nach Anspruch 6 oder Verwendung nach Anspruch 7, wobei das MRT-Kontrastmittel einen Gd³⁺-Komplex einer Verbindung der Formel (I) 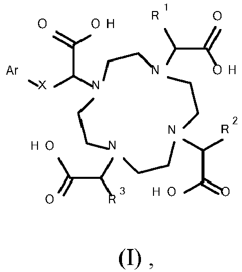 umfasst, wobei
Ar eine Gruppe ausgewählt aus 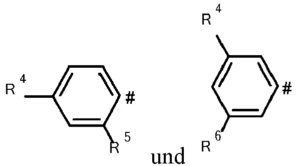 darstellt,
wobei # die Anknüpfung zu X darstellt,
X eine Gruppe darstellt, die aus CH₂, (CH₂)₂, (CH₂)₃, (CH₂)₄ und ^{∗}-(CH₂)₂-O-CH₂-^{#} ausgewählt wird,
wobei ^{∗} die Anknüpfung zu Ar darstellt und ^{#} die Anknüpfung zum Essigsäurerest darstellt,
R¹, R² and R³ unabhängig voneinander ein Wasserstoffatom oder eine Gruppe ausgewählt aus C₁-C₃-Alkyl, -CH₂OH, -(CH₂)₂OH und -CH₂OCH₃ darstellen,
R⁴ eine Gruppe ausgewählt aus C₂-C₄-Alkoxy, (H₃C-CH₂)-O-(CH₂)₂-O-, (H₃C-CH₂)-O-(CH₂)₂-O-(CH₂)₂-O- und (H₃C-CH₂)-O-(CH₂)₂-O-(CH₂)₂-O-(CH₂)₂-O- darstellt,
R⁵ ein Wasserstoffatom darstellt,
und R⁶ ein Wasserstoffatom darstellt,
oder ein Stereoisomer, Tautomer, Hydrat, Solvat oder Salz davon, oder eine Mischung davon oder
wobei das MRT-Kontrastmittel einen Gd³⁺-Komplex einer Verbindung der Formel (II) 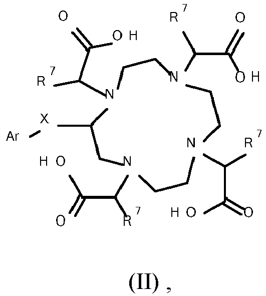
umfasst, wobei
Ar eine Gruppe ausgewählt aus 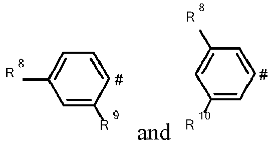 darstellt,
wobei _{#} die Anknüpfung zu X darstellt,
X eine Gruppe darstellt, die aus CH₂, (CH₂)₂, (CH₂)₃, (CH₂)₄ and ^{∗}-(CH₂)₂-O-CH₂-^{#} ausgewählt wird, wobei ^{∗} die Anknüpfung zu Ar darstellt und ^{#} die Anknüpfung zum Essigsäurerest darstellt,
R⁷ ein Wasserstoffatom oder eine Gruppe ausgewählt aus C₁-C₃-Alkyl, -CH₂OH, -(CH₂)₂OH und -CH₂OCH₃ darstellt;
R⁸ eine Gruppe ausgewählt aus C₂-C₄-Alkoxy, (H₃C-CH₂O)-(CH₂)₂-O-, (H₃C-CH₂O)-(CH₂)₂-O-(CH₂)₂-O- und (H₃C-CH₂O)-(CH₂)₂-O-(CH₂)₂-O-(CH₂)₂-O- darstellt;
R⁹ und R¹⁰ unabhängig voneinander ein Wasserstoffatom darstellen;
oder ein Stereoisomer, Tautomer, Hydrat, Solvat oder Salz davon, oder eine Mischung davon.
